Europäisches Patentamt

European Patent Office

Office européen des brevets

(11) Veröffentlichungsnummer : **0 094 644**
**B1**

(12) **EUROPÄISCHE PATENTSCHRIFT**

(45) Veröffentlichungstag der Patentschrift :
30.07.86

(21) Anmeldenummer : **83104746.9**

(22) Anmeldetag : **13.05.83**

(51) Int. Cl.⁴ : **A 61 K 31/59// C07C172/00**

(54) Verwendung eines Cholecalciferolderivates.

(30) Priorität : **17.05.82 US 379384**
**17.05.82 US 379385**

(43) Veröffentlichungstag der Anmeldung :
**23.11.83 Patentblatt 83/47**

(45) Bekanntmachung des Hinweises auf die Patenterteilung : **30.07.86 Patentblatt 86/31**

(84) Benannte Vertragsstaaten :
**BE CH DE FR GB IT LI LU NL SE**

(56) Entgegenhaltungen :
DE-A- 2 021 982
FR-A- 2 453 151
US-A- 3 879 548
US-A- 4 110 446
US-A- 4 201 881
DR. BURGHARD HELWIG et al.: "Moderne Arzneimittel", 5. völlig neubearbeitete Auflage, 1980, Seiten
1441-1446, Wissenschaftliche Verlagsgesellschaft
mbH., Stuttgart, DE.

(73) Patentinhaber : **F. HOFFMANN-LA ROCHE & CO.**
**Aktiengesellschaft**

**CH-4002 Basel (CH)**

(72) Erfinder : **Boris, Alfred**
**25 Lord Sterling Drive**
**Parsippany, N.J. (US)**
Erfinder : **Partridge, John Joseph**
**121 Norwood Avenue**
**Upper Montclair, N.J. (US)**
Erfinder : **Uskokovic, Milan Radoje**
**252 Highland Avenue**
**Upper Montclair, N.J. (US)**

(74) Vertreter : **Lederer, Franz, Dr. et al**
**Patentanwälte Dr. Lederer Franz Meyer-Roxlau Rei-**
**ner F. Lucile-Grahn-Strasse 22**
**D-8000 München 80 (DE)**

# 0 094 644

**Beschreibung**

Die Erfindung beruht auf dem Auffinden von neuen physiologischen Eigenschaften des 24,24-Difluor-$1\alpha$,25-dihydroxycholecalciferols, das weiter unten die fluorierte Verbindung genannt wird. Insbesondere wurde gefunden, dass die fluorierte Verbindung gegen Osteoporose und bei der Verhütung des Milchfiebers wirksam ist. Die fluorierte Verbindung kann daher bei der Behandlung der Osteoporose durch Verabreichung einer effektiven Dosis der fluorierten Verbindung an einen an dieser Krankheit leidenden Patienten sowie bei der präventiven Behandlung des Milchfiebers durch Verabreichung einer effektiven Dosis fluorierten Verbindung an einen trächtigen Wiederkäuer verwendet werden.

Aus US-A-4201881 und FR-A-2453151 ist bekannt, dass man 24,24-Difluor-$1\alpha$,25-dihydroxycholecalciferol(nachstehend 24,24-$(F)_2$-$1\alpha$,25-$(OH)_2$-$D_3$ bezeichnet) auf Grund seiner Vitamin D-Aktivität (Stimulierung des intestinalen Calcium-Transportes, Mobilisierung des Calciums aus den Knochen) als Arzneimittel verwenden kann.

Die Erfindung betrifft die Verwendung der fluorierten Verbindung bei der Herstellung eines Mittels zur Behandlung der Osteoporose und des Milchfiebers, sowie pharmazeutische Präparate, die etwa 30-500 $\mu$g der fluorierten Verbindung in Kombination mit einem pharmazeutisch verwendbaren inerten Trägermaterial zur präventiven Behandlung des Milchfiebers enthalten.

Die obigen Aktivitäten können in den folgenden Versuchen nachgewiesen werden :

a) Antirachitische Aktivität

Weissen Leghorn-Küken wird unter UV-freier Beleuchtung eine Vitamin D-arme, 1 % Calcium und 0,7 % Phosphor enthaltende Diät gegeben. Die in Propylenglykol gelöste Testverbindung wird während 21 Tagen den am Anfang des Versuchs 1 oder 2 Tage alten Küken oral verabreicht. Kontrolltiere werden mit dem Vehikel behandelt. Die Küken werden am Tag nach dem letzten Behandlungstag seziert und das Tibiaascchengewicht wird ermittelt. Es werden 9 oder 10 Küken für jede Behandlungsgruppe und für die Kontrollgruppe verwendet. Die mittleren Tibiaaschengewichte ausgedrückt in mg sind in der nachfolgenden Tabelle I angegeben. Die Resultate zeigen, dass die fluorierte Verbindung eine beträchtliche antirachitische Aktivität aufweisen.

Tabelle I

| Dosis (ng/Küken/Tag) | Mittleres Tibiaaschengewicht (mg) |
|---|---|
| 0 | 120,7 $\pm$ 5,9 |
| 1 | 111,5 $\pm$ 4,7 |
| 3 | 151,1 $\pm$ 8,2 |
| 10 | 227,1 $\pm$ 8,2 |
| 30 | 244,8 $\pm$ 7,4 |

b) Intestinale Calcium-Absorption bei Küken

Weissen Leghorn-Küken wird während 21 Tagen unter UV-freier Bestrahlung eine Vitamin D-arme Diät gegeben. Eine Einzeldosis der Testverbindung, gelöst in Propylenglykol, wird den Tieren oral verabreicht. Danach werden zu verschiedenen Zeitpunkten 2 $\mu$Ci $^{45}$Ca (Chlorid) oral verabreicht und die Serumradioativität 45 Minuten nach verabreichung des Isotops gemessen. In jeder Versuchsperiode werden Kontrolltiere verwendet, denen nur das Vehikel verabreicht wird. In jeder Behandlungsgruppe und jeder Kontrolgruppe werden 10 oder 11 Küken verwendet. Die in der Tabelle II für $1\alpha$,25-Dihydroxycholecalciferol (Verbindung A) und für die fluorierte Verbindung (B) gegebenen Resultate zeigen, dass letztere eine lang andauernde intestinale Calcium-Absorption (96 Stunden nach 100 ng Einzeldosis) bewirkt und daher bei der Verhütung des Milchfiebers bei trächtigen Wiederkäuern verwendbar ist.

(Siehe Tabelle II Seite 3 f.)

2

**0 094 644**

Tabelle II

| Behandlung | Zeit (Std.) | Anzahl Küken | Serum $^{45}$Ca (cpm/0,2 ml) |
|---|---|---|---|
| Vehikel 0,2 ml | 24 | 11 | 992 ± 81 |
| Verbindung A 100 ng | | 11 | 1800 ± 181 |
| Verbindung B 100 ng | | 11 | 2064 ± 170 |
| Vehikel 0,2 ml | 48 | 11 | 769 ± 90 |
| Verbindung A 100 ng | | 11 | 1006 ± 133 |
| Verbindung B 100 ng | | 11 | 1539 ± 99 |
| Vehikel 0,2 ml | 72 | 10 | 647 ± 69 |
| Verbindung A 200 ng | | 11 | 710 ± 62 |
| Verbindung B 100 ng | | 11 | 1164 ± 90 |
| Vehikel 0,2 ml | 96 | 10 | 586 ± 70 |
| Verbindung B 100 ng | | 10 | 998 ± 61 |

c) Verhütung der durch EHDP-induzierten Blockierung der Mineralisierung bei Ratten

Männliche Charles River Ratten werden 10 Tage mit Dinatrium-äthan-1-hydroxy-1,1-diphosphonat (EHDP) behandelt. Die Verbindung wird in destilliertem Wasser in einer Konzentration von 2 mg/0,2 mg/Ratte jeden Tag subcutan verabreicht. Die fluorierte Verbindung wird in Propylenglykol jeden Tag oral verabreicht. Am Tag nach der letzten Behandlung werden die Ratten seziert und deren Tibia durch Silberimprägnierung der Knochensalze behandelt. Die Breite der Epiphysenplatte wird mit einem Mikroskop gemessen. Zur Ermittlung der Aktivität wird die dosisabhängige Verengerung der durch EHDP-Induktion erweiterten Epiphysenplatte gemessen. In jeder Berhandlungsgruppe werden 10 Ratten verwendet. Es werden auch positive (EHDP allein) und negative (Vehikel allein) Kontrollgruppen von 10 Ratten in jedem Versuch verwendet. Die in der Tabelle III angegebenen Resultate zeigen, dass die fluorierte Verbindung die Calcifizierung der Tibiaepiphysenplatte bei EHDP-blockierten Ratten bewirkt.

Tabelle III

| Dosis (ng/Ratte/Tag) | Mittlere Breite der Epiphysenplatte (Mikron) |
|---|---|
| 0 | 1182 ± 51 |
| 1 | 839 ± 18 |
| 3 | 674 ± 18 |
| 10 | 540 ± 16 |
| 30 | 412 ± 9 |
| Vehikel-Kontrollen (kein EHDP) | 440 ± 2 |

Die fluorierte Verbindung kann in Tagesdosen im Bereich von etwa 50-200, vorzugsweise 100 ng zur Behandlung der Osteoporose verabreicht werden. Sie werden vorzugsweise oral, z. B. in Form von Tabletten, Kapseln oder Elixieren verabreicht, können aber auch parenteral, insbesondere subcutan, intramuskulär, intravenös oder intraperitoneal, z. B. in Form von sterilen Lösungen oder Suspensionen, verarbreicht werden. Etwa 50 bis 200 ng der fluorierten Verbindung werden mit pharmazeutisch

3

anwendbaren Hilfsstoffen, wie Vehikeln, Trägern, Excipientien, Bindemitteln, Konservierungsmitteln, Stabilisierungsmitteln und/oder Geschmackstoffen, in einer Einzeldosis vermischt. Beispiele von Hilfsmittel, die in Kapseln verwendet werden können, sind Bindemittel, wie Maisstärke oder Gelatine ; Excipientien, wie Dicalciumphosphat ; Zersetzungsmittel, wie Mais- oder Kartoffelstärke oder Alginsäure ; Gleitmittel, wie Magnesiumstearat ; Süssstoffe, wie Sucrose ; Geschmacksmittel, wie Pfefferminze. Es können andere Materialien, wie Ueberzüge zur Modifizierung des Aspektes der Einzeldosen, verwendet werden. Tabletten können z. B. mit Schellack, Zucker oder beide überzogen werden. Ein Sirup oder Elixier kann die Aktivsubstanz, Sucrose als Süssmittel, Methyl- und Propylparaben als Konservierungsmittel, einen Farbstoff und einen Geschmackstoff, wie Orangearoma, enthalten. Sterile Präparate zum Injizieren können in üblicher Weise dadurch hergestellt werden, dass man die fluorierte Verbindung in einem Vehikel, wie Injektionswasser, einem in der Natur vorkommenden pflanzlichen Oel, wie Sesamöl, oder einem synthetischen fetten Bindemittel, wie Aethyloleat, auflöst oder suspendiert. Es können auch Puffer, Konservierungsmittel und Antioxidantien zugesetzt werden.

Die fluorierte Verbindung kann in Tagesdosen im Bereich von etwa 30-500 µg zur Verhütung des Milchfiebers trächtigen Wiederkäuern, insbesondere trächtigen Kühen, vorzugsweise ein bis vier Tagen vor der Niederkunft, verabreicht werden; Die fluorierte Verbindung kann in Mengen von 30-500 µg in Fettsäuregranulaten zur oralen Verabreichnung eingefügt werden. Sterile Präparate zum Injizieren und/oder zur topischen Verabreichung können in üblicher Weise dadurch hergestellt werden, dass man die fluorierte Verbindung in einem Vehikel, wie einem 5-10 %igen Aethanol-Wasser-Gemisch, einem in der Natur vorkommenden pflanzlichen Oel, z. B. Sesamöl, einem synthetischen fetten Bindemittel, z. B. Aethyloleat, auflöst oder suspendiert. Zum Beispiel kann ein Präparat zum intravenösen Injizieren aus 2-3 ml einer 5-10 %igen Aethanol-Wasser-Lösung enthaltend 30-500 µg, vorzugsweise 30-400 µg, der fluorierten Verbindung bestehend. Ein Präparat zur topischen Verabreichung kann aus einer pflanzlichen Oellösung oder Oelsuspension enthaltend 30-500 µg, vorzugsweise 250-500 µg, der fluorierten Verbindung bestehen. Die fluorierte Verbindung kann auch in ein Präparat zum intramuskulären Injizieren eingefügt werden, indem man 50-500 µg der fluorierten Verbindung in einem Vehikel, wie einem 5-10 %igen Aethanol-Wasser- oder Propylenglykol-Wasser-Gemisch suspendiert. Es können auch Puffer, Konservierungsmittel und/oder Antioxidantien zugesetzt werden.

Beispiel

Ein Kapsel bestehend aus

| | | | | | | | |
|---|---|---|---|---|---|---|---|
| 1. der fluorierten Verbindung | 50 | ng | 100 | ng | 200 | ng |
| 2. Polyäthylenglykol 400 | 200 | mg | 200 | mg | 200 | mg |
| 3. butyliertem Hydroxyanisol | 0,1 | mg | 0,1 | mg | 0,1 | mg |
| 4. Ascorbylpalmitat | 1,0 | mg | 1,0 | mg | 1,0 | mg |

kann dadurch hergestellt werden, dass man die Bestandteile 1, 3 und 4 in den Bestandteil 2 unter Stickstoffatmosphäre auflöst und in Kapseln abfüllt.

**Patentansprüche**

1. Die Verwendung des 24,24-Difluor-1α,25-dihydroxycholecalciferols bei der Herstellung eines Mittels zur Behandlung der Osteoporose.

2. Die Verwendung des 24,24-Difluor-1α,25-dihydroxycholecalciferols bei der Herstellung eines Mittels zur präventiven Behandlung des Milchfiebers.

3. Präparate enthaltend etwa 30 bis 500 µg 24,24-Difluor-1α,25-dihydroxycholecalciferol in Kombination mit einem pharmazeutisch verwendbaren inerten Trägermaterial zur präventiven Behandlung des Milchfiebers.

**Claims**

1. The use 24,24-difluoro-1α,25-dihydroxycholecalciferol in the manufacture of a medicament for the treatment of osteoporosis.

2. The use 24,24-difluoro-1α,25-dihydroxycholecalciferol in the manufacture of a medicament for the preventative treatment of milk fever.

3. Preparations containing about 30 to 500 µg of 24,24-difluoro-1α,25-dihydroxycholecalciferol in combination with a pharmaceutically usable inert carrier material for the preventative treatment of milk fever.

**0 094 644**

**Revendications**

1. L'utilisation du 24,24-difluoro-1 alpha, 25-dihydroxycholécalciférol dans la préparation d'un produit pour le traitement de l'ostéoporose.

2. L'utilisation du 24,24-difluoro-1 alpha, 25-dihydroxycholécalciférol dans la préparation d'un produit pour le traitement préventif de la fièvre du lait.

3. Compositions contenant d'environ 30 à 500 microgrammes de 24,24-difluoro-1, alpha, 25-dihydroxycholécalciférol en combinaison avec un véhicule inerte convenant pour l'usage pharmaceutique pour le traitement préventif de la fièvre du lait.